# EUROPEAN PATENT APPLICATION

(11) **EP 4 734 124 A1**
(43) Date of publication of application: **29.04.2026**
(21) Application number: 25209321.6
(22) Date of filing: 17.10.2025
(51) Int. Cl.: G16H 50/20, A61B 5/11, G16H 50/30

(54) **DIGITAL SPIRAL TRACING AND FREE DRAWING TESTS**

(30) Priority: 22.10.2024 US 202463710470 P
(71) Applicant: Linus Health, Inc., Boston, MA 02210 (US)
(72) Inventor: HIGGINS, Connor, Boston (US); BANKS, Russell, Boston (US); CONNER, Marissa Ciesla, Boston (US); CHEN, Emma, Boston (US); JANNATI, Ali, Boston (US); SUH, Abigail, Boston (US); BATES, David, Boston (US); PASCUAL-LEONE, Alvaro, Boston (US)
(74) Representative: Müller-Boré & Partner Patentanwälte PartG mbB

(57) **Abstract**

Systems and methods are directed to screening a subject for motor and cognitive impairment. A method for screening motor and cognitive impairment comprises administering a spiral tracing assessment to a subject, and receiving a response thereto; assessing the subject's performance based on the response; collecting performance parameters based on the subject's performance on the spiral tracing assessment; providing the performance parameters and the response to a pretrained machine learning model; predicting, using the pretrained machine learning model, a likelihood of the patient having a cognitive impairment; determining a motor and cognitive impairment status of the patient based on the prediction; and outputting the motor and cognitive impairment status.

## Description

### RELATED APPLICATION(S)

This application claims the benefit of priority to U.S. Provisional App. No. 63/710,470, filed October 22, 2024, which is herein incorporated by reference in its entirety.

### TECHNICAL FIELD

Embodiments of the present disclosure are related to a system, method, and computer program product for a neuropsychological assessment.

### BACKGROUND OF THE DISCLOSURE

Paper and pencil Digital Spiral Tracing and Free Drawn Spiral Tests have been used to detect tremor and other motor and cognitive concerns. However, administration and interpretation of this test in the clinical setting is time consuming and subjective to the scorer's interpretation.

What is needed is a digital adaptation of the Spiral Tracing and Free Drawing assessment by incorporating digital stylus metrics, tablet based data storage, and algorithmic feature extraction and analytics to detect motor disorders, such as tremor and parkinsonism (micrographia and shaky drawing), stroke (limb apraxia and dystonia), spinal muscular atrophy, Huntington's Disease, and other upper motor neuron disease processes. A digital assessment provides automatic administration, machine learning and AI processing, scoring, and interpretation which saves time in the busy clinical environment. Additionally, the digital Spiral Free Drawing solution offers personalized recommendations based on the individual's test outcomes.

### SUMMARY

Here, a Digital Spiral Tracing and Free Drawn Spiral Test is disclosed.

In one embodiment, a method for screening motor and cognitive impairment comprises administering a spiral tracing assessment to a subject, and receiving a response thereto; assessing the subject's performance based on the response; collecting performance parameters based on the subject's performance on the spiral tracing assessment; providing the performance parameters and the response to a pretrained machine learning model; predicting, using the pretrained machine learning model, a likelihood of the patient having a cognitive impairment; determining a cognitive impairment status of the patient based on the prediction; and outputting the motor and cognitive impairment status.

In some embodiments, the spiral tracing assessment comprises directing the subject to trace a spiral in a clockwise and/or anti-clockwise direction.

In some embodiments, the spiral tracing assessment comprises directing the subject to trace a spiral with a dominant and/or non-dominant hand.

In some embodiments, the spiral tracing assessment comprises directing the subject to trace a spiral with one continuous pen stroke.

In some embodiments, the spiral tracing assessment is completed with a digital pen.

In some embodiments, collecting performance parameters comprises reading one or more digital pen metrics, the one or more digital pen metrics collected during the spiral tracing assessment.

In some embodiments, the response to the spiral tracing assessment comprises one or more traced spirals.

In some embodiments, the method further comprises determining one or more metrics based on one or more deviations between a template spiral and the one or more traced spirals.

In some embodiments, the method further comprises combining the one or more metrics into a composite score; comparing the composite score to a threshold value; and based on the comparison, determining a motor and cognitive impairment level of the subject.

In some embodiments, the motor and cognitive impairment status is one of mild or moderate.

In some embodiments, the method further comprises suggesting additional testing based on the output motor and cognitive impairment status.

In some embodiments, the motor and cognitive impairment comprises a tremor.

In some embodiments, the tremor is determined by one or more graphomotor metrics.

In some embodiments, the tremor is determined by one or more metrics derived from a digital pen.

In some embodiments, administering a spiral tracing assessment to a subject comprises providing the spiral tracing assessment to the subject on a tablet.

In some embodiments, the method further comprises allowing the subject to complete one or more practice spiral tracing assessments without collecting any metrics.

In some embodiments, predicting a likelihood of the patient having a cognitive impairment comprises predicting a degree of cognitive impairment.

In some embodiments, the degree of cognitive impairment comprises one of mild, moderate, or severe.

In an alternate embodiment, a method, in particular for screening motor and cognitive impairment and/or for determining and outputting a motor and cognitive impairment status, comprises receiving a response to a spiral tracing assessment administered to a subject or receiving data comprising a response to a spiral tracing assessment administered to a subject; assessing the subject's performance based on the response; collecting performance parameters based on the subject's performance on the spiral tracing assessment; providing the performance parameters and the response to a pretrained machine learning model; predicting, using the pretrained machine learning model, a likelihood of the patient having a cognitive impairment; determining a motor and cognitive impairment status of the patient based on the prediction; and outputting the motor and cognitive impairment status.

In some embodiments, the method can further comprise administering a spiral tracing assessment to a subject, in particular prior to receiving the response of a spiral assessment administered to a subject.

The method can comprise the features of the embodiments of the preceding method.

In an alternate embodiment, a system for screening motor and cognitive impairment comprises a computing node comprising a computer readable storage medium having program instructions embodied therewith, the program instructions executable by a processor of the computing node to cause the processor to perform a method comprising: administering a spiral tracing assessment to a subject, and receiving a response thereto; assessing the subject's performance based on the response; collecting performance parameters based on the subject's performance on the spiral tracing assessment; providing the performance parameters and the response to a pretrained machine learning model; predicting, using the pretrained machine learning model, a likelihood of the patient having a cognitive impairment; determining a cognitive impairment status of the patient based on the prediction; and outputting the cognitive impairment status.

In an alternate embodiment, a computer program product for screening cognitive impairment, the computer program product comprising a computer readable storage medium having program instructions embodied therewith, the program instructions executable by a processor to cause the processor to perform a method comprising: administering a spiral tracing assessment to a subject, and receiving a response thereto; assessing the subject's performance based on the response; collecting performance parameters based on the subject's performance on the spiral tracing assessment; providing the performance parameters and the response to a pretrained machine learning model; predicting, using the pretrained machine learning model, a likelihood of the patient having a cognitive impairment; determining a cognitive impairment status of the patient based on the prediction; and outputting the cognitive impairment status.

### BRIEF DESCRIPTION OF THE DRAWINGS

The accompanying drawings, which are incorporated into and constitute a part of this specification, illustrate various exemplary embodiments and together with the description, serve to explain the principles of the disclosed embodiments.
**FIG. 1** is a screen display of a spiral drawing test using checkpoint measurements.
**FIG. 2** is a flowchart of an exemplary method for a digital spiral drawing test, according to embodiments disclosed herein.
**FIG. 3** is a sample of a hand-drawn spiral test for a spiral crossing task.
**FIG. 4** is an illustration of an exemplary instruction screen, according to embodiments disclosed herein.
**FIG. 5** is a series of illustrations of instruction screens with a corresponding example animation, according to embodiments disclosed herein.
**FIG. 6** is an illustration of an exemplary practice screen, according to embodiments disclosed herein.
**FIG. 7** is an illustration of an exemplary instruction screen for how to hold an instrument during an assessment, according to embodiments disclosed herein.
**FIG. 8** is an illustration of an exemplary instruction screen for drawing a first spiral, according to embodiments disclosed herein.
**FIG. 9** is an illustration of an exemplary instruction screen for drawing an additional spiral, according to embodiments disclosed herein.
**FIG. 10** is an illustration of an exemplary instruction screen for how to adjust the instrument during the assessment, according to embodiments disclosed herein.
**FIG. 11** is an illustration of an exemplary instruction screen for drawing another spiral, according to embodiments disclosed herein.
**FIG. 12** is an illustration of an exemplary instruction screen for drawing a final spiral, according to embodiments disclosed herein.
**FIG. 13** is an exemplary computing node.

### DETAILED DESCRIPTION

Reference will now be made in detail to the exemplary embodiments of the present disclosure, examples of which are illustrated in the accompanying drawings. Wherever possible, the same reference numbers will be used through-out the drawings to refer to the same or like parts.

The systems, devices, and methods disclosed herein are described in detail by way of examples and with reference to the figures. The examples discussed herein are examples only and are provided to assist in the explanation of the apparatuses, devices, systems, and methods described herein. None of the features or components shown in the drawings or discussed below should be taken as mandatory for any specific implementation of any of these devices, systems, or methods unless specifically designated as man-datory.

Also, for any methods described, regardless of whether the method is described in conjunction with a flow diagram, it should be understood that unless otherwise specified or required by context, any explicit or implicit ordering of steps performed in the execution of a method does not imply that those steps must be performed in the order presented but instead may be performed in a different order or in parallel.

As used herein, the term "exemplary" is used in the sense of "example," rather than "ideal." Moreover, the terms "a" and "an" herein do not denote a limitation of quantity, but rather denote the presence of one or more of the referenced items.

This application discloses systems and techniques for screening or diagnosing cognitive impairment, possibly in conjunction with detection and/or quantification of patient tremor. One embodiment may require a subject to draw, for example, a spiral using a digitizing pen, although other drawing, writing, and pen stroke capture techniques may be utilized. Some tests may ask the subject to follow a template spiral or other assigned pattern while holding his/her arm off the table, while other tests disclosed herein may allow the subject to hold the digitizing pen comfortably and write freehand on a sheet of paper.

Features derived from the recorded data may be processed by algorithms into scores that correlate with different types of cognitive impairment. Cognitive impairment can be inferred by incorrect positioning. Techniques may further include using a digitizing pen that records its position on the page as a function of time. Many more features can be accurately measured in this manner, such as pauses between drawing different portions of the spiral, timing of strokes, precise positioning of features relative to the nominal center of the drawing, and so forth. Using the digital spiral data, classifiers may be created that function either as screening or diagnostic tests for multiple types of cognitive impairment.

Another neurological disorder that may be monitored is tremor, especially as this is often associated with serious medical conditions such as Parkinson's disease. Some methods of measuring tremor may require subjects to use a biomedical sensor such as an accelerometer.

In addition, the present analysis, according to exemplary embodiments, may derive low-pass, bandpass velocity, and bandpass acceleration measures and components of those entities in specific directions, possibly related to the direction of pen motion, any of which may be derived from one or more pen strokes within the drawing. These measures may be used to compute several robust tremor quantification features that correlate strongly with tremor. Although the Digital Clock Test is described in connection with many of the embodiments, other types of tests and drawings may be used and fall within the scope of the inventions.

Neuropsychological assessment tools are traditionally applied using paper and pencil to evaluate motor skills, visual-motor integration, and cognitive function. These tools are used in clinical settings, particularly in assessments of neurological conditions and cognitive impairments where primary deficits manifest in motor impairment. Digital spiral tracing and free drawn spiral tests can provide valuable information about an individual's hand-eye coordination, motor control, attention to detail, and spatial awareness. Deviations from the spiral path or irregularities in tracing indicate motor skill deficits, visual-motor integration problems, or other cognitive impairments related to neurodegeneration or brain injury. They are often used as part of a battery of tests to gain a comprehensive understanding of an individual's cognitive and motor abilities.

Digital spiral tracing and free drawn spiral tests differ from other digital tests in that they do not require the subject to draw complex figures with a complicated series of drawing strokes. For example, some alternative digital solutions may require the subject to draw a figure such as a clock set to a specific time. In contrast, embodiments of the present disclosure use a simple continuous stroke to reveal subtle motor effects due to its continuous nature and the need to keep the stylus engaged with the tablet, as opposed to repeatedly lifting the pen or stylus to draw non-continuous elements.

Alternative solutions for the spiral tracing tests limit adaptations of the paper and pencil based test. For example, one alternate solution is using a finger-based drawing assessment. In this solution, the assessment relies on the finger to draw shapes, lacking metrics derived from digital stylus use and integration.

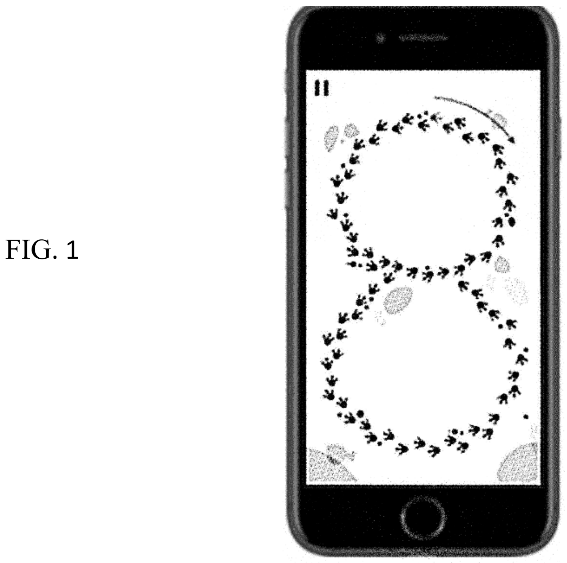

The majority of alternative solutions using spiral tracing measures time and stability between "checkpoints", as illustrated in **FIG. 1****.** In contrast, embodiments of the present disclosure measure the deviation from a target spiral, time to completion, stroke count, pixel length of each stroke, distance of each stroke and total drawing stroke, laterality of stroke to complete the spiral (*i.e.,* left vs. right vs. superior vs. inferior concentration of stroke), *etc.* Further, the embodiments disclosed herein are digital, as opposed to the finger tracing of the spiral shape in alternative solutions.

Digital spiral tracing and free drawing tests allow for standardized administration and unbiased, objective scoring and interpretation that may be varied during traditional paper and pencil-based or finger-based administration. The digital spital tracing and free drawing solution is more sensitive in detecting subtle motor and cognitive concerns, possesses more sensitivity across populations, and is less subjective in scoring, eliminating chances of subjective interpretation. Further, by incorporating the metrics collected from the digital stylus, data unavailable in previous contexts is incorporated into the processing and algorithmic classification of impairment. Additionally, embodiments of the digital spiral tracing and free drawing tests may provide personalized recommendations provided to the individual based on the test results.

Embodiments of the digital spiral tracing and free drawing tests may be utilized by a range of users. Neurology specialists or researchers wishing to assess motor function characteristics may include the tests in cognitive impairment assessments in a clinical setting. Practice managers, or those managing the operational aspects of a clinic or practice, may also benefit from use of the tests, as the digital tests require fewer materials for application. Clinicians, such as MDs, DOs, NPs, PAs, RNs, MAs, OTs, or SLPs, may use the tests in cognitive applications. In some settings, patients (*i.e.*, users being assessed for cognition and brain health) age 55 and older may be able to apply and use the tests themselves. For example, patients in a community or in an assistive facility, and may visit clinics for regular or acute checkups. Additionally, primary site contacts in manager roles may use the tests, especially in cases where the site contacts have responsibility across multiple sites and may want to view patient information and results across many clinic locations within a parent organization.

Embodiments of the present disclosure are delivered on a digital platform and automatically scored, reducing the need for scarce resources and skilled personnel effort. This also allows embodiments of the disclosure to be universally available to anyone with digital access to the test platform and takes substantially less time to complete than a traditional assessment. Another advantage of the proposed technique is that it does not rely on information obtained from an informant. Accordingly, embodiments of the present disclosure reduce the need for additional personnel needed to complete cognitive screening and can indicate those at higher risk for cognitive impairments.

Some embodiments of the digital spiral tracing and free drawing test are administered on a tablet device with the use of a Bluetooth stylus. The test may use a React Native web app to capture touch screen drawing data via mobile application and a pressure and force sensitive stylus. Graphomotor metrics data may be transmitted to a cloud infrastructure and processed using a specific algorithm for each digital spiral tracing and drawing test. Specific algorithms may be used to calculate metrics, such as pen velocity, number of strokes, and stroke lengths. Some algorithms may be used to calculate metrics such as distance in pixels or deviation from an ideal spiral. Processed graphomotor metrics may be transmitted and stored in a cloud infrastructure in some embodiments, with results displayed in a web-based dashboard.

In some embodiments, the application software may also provide user detailed analysis of the subject's test performance, including parameters related to test performance across for each sub-task against a reference population. In some embodiments, the application software may create provider and patient-facing reports, which may include recommendations at the end of each valid test that the user can review or print.

Graphometer metrics may include, but are not limited to:
- Total time to complete the given spiral;
- Total number of strokes used to complete the given spiral;
- Total length in pixels of the drawn spiral;
- Mean distance in pixels that the drawn spiral was to the trace spiral;
- Maximum distance in pixels that the drawn spiral was to the trace spiral;
- Standard deviation of the distance in pixels that drawn spiral was to the trace spiral;
- Mean distance in pixels that left half of the drawn spiral was to the left half of the trace spiral;
- Mean distance in pixels that right half of the drawn spiral was to the right half of the trace spiral;
- Maximum distance in pixels that left half of the drawn spiral was to the left half of the trace spiral;
- Maximum distance in pixels that right half of the drawn spiral was to the right half of the trace spiral;
- The standard deviation of the distance in pixels that the left half of the drawn spiral was to the left half of the trace spiral; and
- The standard deviation of the distance in pixels that the right half of the drawn spiral was to the right half of the trace spiral

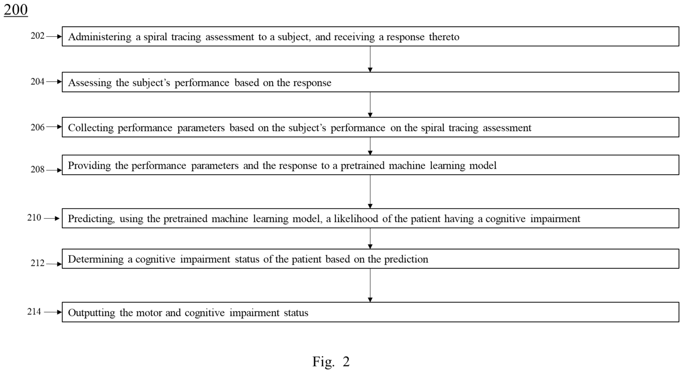

**FIG. 2** is a flowchart of an exemplary method **200** for a determining a motor and cognitive impairment status using a digital spiral tracing test. For example, the method **200** (*i.e.,* steps **202-214)** may be performed by a test device automatically or in response to a request from a user *(e.g.,* a clinician, administrator, *etc.).*

According to some embodiments, the method **200** may include one or more of the following steps. In step **202,** the method may include administering a spiral tracing assessment to a subject, and receiving a response thereto. The spiral tracing assessment comprises directing the subject to trace a spiral in a clockwise and/or anti-clockwise direction, with a dominant or non-dominant hand, and to trace the spiral with one continuous stroke. The spiral tracing assessment is to be completed using a digital pen, such as a Bluetooth stylus. The motor and cognitive impairment can include, for example, a tremor, as determined by one or more graphomotor metrics. The spiral tracing assessment may be administered on a tablet, smart phone, or other suitable device.

In step **204,** the method may include assessing the subject's performance based on the response. The response comprises the spiral pens traced by the subject, and includes metrics based on deviations from the template spiral and the traced spiral. The one or more metrics are comprised into a composite score for the spiral tracing assessment. This composite score can be compared to a threshold value to determine a motor and/or cognitive impairment level of the subject. The motor and/or cognitive impairment level includes mild, moderate, or in cases where no impairment is detected, not applicable. The pen-derived metrics may be used to indicate the presence of a tremor.

In step **206,** the method may include collecting performance parameters based on the subject's performance on the spiral tracing assessment. Performance parameters may include, but are not limited to timing, pen-derived metrics related to motor skills and drawing efficiency, including but not limited to a force measurement, a directional measurement, a pressure measurement, and an altitude measurement. Exemplary metrics are defined in **Table 1,** below:

**Table 1: Exemplary Metrics**

| **Metric** | **Definition** |
|---|---|
| Stylus Oscillatory Motion | A measure of how much the motion of the pen deviates from a smooth pen motion during the drawing process of the copying of the spiral. |
| Stylus Pressure | The force of the touch, where a value of 1 represents the force of an average touch, a predetermined benchmark set by the stylus system. The force reported is measured along the axis of the pencil and may be included into a deep learning model that produces a prediction for each subject. |
| Objective Feature: Azimuth | A measure of the direction in which the stylus is pointing in radians relative to the plane of the screen. The azimuth angle increases as the user swings the cap-end of the stylus in a clockwise direction around the tip and may be included into a deep learning model that produces a prediction for each subject. |
| Objective Feature: Altitude | A measure of the elevation of the stylus above the plane of the screen in radians. A value of 0 radians indicates that the stylus is parallel to the surface, which increases as the user lifts the cap-end from the surface and may be included into a deep learning model that produces a prediction for each patient. |
| Objective Feature: Stroke Count | The number of individual pen strokes produced in the drawing of either the command or copy clock, with each stroke delineated by each stylus point-end lift from the tablet surface and may be incorporated into a deep learning model that produces a prediction for each subject. |

In some embodiments, the method may include allowing the subject to practice drawing the spiral before recording or collecting any metrics or data.

In step **208,** the method may include providing the performance parameters and the response to a pretrained machine learning model. The performance parameters are collected by reading one or more digital pen metrics communicated by the digital pen, and collected during the subject's completion of the spiral tracing assessment.

In step **210,** the method may include predicting, using the pretrained machine learning model, a likelihood of the patient having a cognitive impairment. The likelihood of the subject having a cognitive impairment includes a prediction of a degree of cognitive impairment, where the degree is one of mild, moderate, or severe.

In step **212,** the method may include determining a cognitive impairment status of the patient based on the prediction.

In step **214,** the method may include outputting the motor and cognitive impairment status. Based on this status, the method may include suggesting additional testing.

In a standard spiral tracing test, the subject traces an anti-clockwise and a clockwise spiral with their dominant hand, as well as an anti-clockwise and a clockwise spiral with their non-dominant hand on a tablet device using a Bluetooth stylus. A time limit may be implemented, where the subject must complete the drawing within the limit. Alternatively, the drawing created within the time limit may be considered complete for the subject. The time limit may be, for example, 60 seconds in which to trace each spiral. Three variants of this test may be included in embodiments of the present disclosure. These variants may include spiral tracing, spiral copy, and spiral crossing.

In various embodiments, a spiral tracing test comprises a patient tracing a spiral in both clockwise and anti-clockwise directions with their dominant and non-dominant hands.

In spiral tracing, the subject is directed to trace a spiral represented by hashed lines on a display screen. In some embodiments, a protocol may include a practice spiral drawing followed by each hand drawing an anti-clockwise (left) and clockwise (right) spiral. The first drawing may be performed using the dominant hand, and the second, clockwise drawing may be performed using the non-dominant hand.

In the spiral copy variant, the subject is directed to draw a spiral based on a reference image located in an upper part of the display screen. A protocol may call for spiral drawing with the dominant hand, followed by a second spiral drawing with the non-dominant hand. Different from spiral tracing, the subject must draw a new spiral that is a copy of the displayed reference spiral.

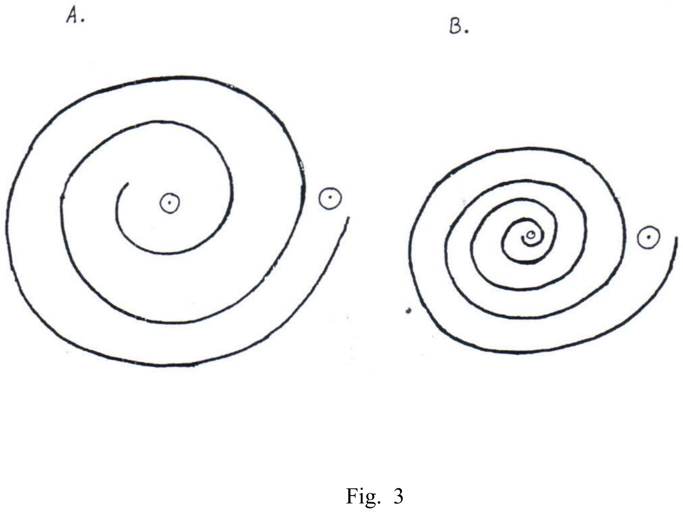

For spiral crossing, a subject may be instructed to draw a line from a starting point to an end point without crossing the lines of a spiral. A protocol may include calling for the dominant hand to be used, and may start with a larger spiral then followed by the same instructions over a smaller spiral. A sample of hand-drawn spiral test for spiral crossing task is provided in **FIG. 3****.** The task is to draw a spiral inside the drawn spiral starting with the inner circle and ending with the outer circle

### Spiral Free Drawing Test

As instructed, the subject draws a spiral based on a reference image displayed in an upper half of a display screen. An associated protocol may call for the subject to draw the spiral with the dominant hand, followed by a second spiral drawing by the non-dominant hand.

In some embodiments, the reference spiral may fill a quarter of a standard letter sized paper. The lines of the spiral may be about 1.3 cm (0.5 in) apart.

In some embodiments, the subject may be instructed to hold the stylus or digital pen such that no part of the subject's upper limb touches the table or tablet for the duration of the test. A clinician or test administrator may assess the feasibility of the set up, where some parameters of the test may need to be adjusted for subject comfort. In some cases, stylus or digital pen practice instructions may need to be modified, as some digital pen instructions explicitly state it is acceptable to contact a surface with a hand or upper limb while using the digital pen.

### Scoring of Spiral Tasks

The standard spiral tracing test may follow the TETRAS tremor scale. The TETRAS scale includes spiral drawing tasks as well as handwriting and dot approximation tasks. The TETRAS scoring specifies that participants should "Demonstrate how to draw Archimedes spiral that approximately fills ¼ of an unlined page of standard (letter) paper. The lines of the spiral should be approximately 1.3 cm (0.5 inch) apart. Then ask the subject to copy the spiral. Test and score each hand separately. Use a ballpoint pen. The pen should be held such that no part of the limb touches the table. Secure the paper on the table in a location that is suitable for the individual's style of drawing. Score the tremor in the spiral, not the movement of the limb."

Scoring of the Archimedes spiral with each hand may be scored as follows:
- 0 = normal
- 1 = slight: tremor barely visible.
- 2 = mild: obvious tremor
- 3 = moderate: portions of figure not recognizable.
- 4 = severe: figure not recognizable

Some embodiments of the present disclosure may provide the following functions. A touchscreen may capture drawing metrics via a digital pen or Bluetooth stylus from an application running as a mobile application on a device. The device may be a tablet, smartphone, or any other suitable device with a processor capable of running the application. In some embodiments, the application may provide a quantitative assessment of the subject's performance in completing the test. This may include an interpretation of the subject's performance in completing the test, which may be displayed as an output task performance metrics and completed spiral free drawing in a web dashboard. In some embodiments, the application may create a caregiver or administrator-facing report at the end of each test, which may be reviewed or printed. The output may include individualized recommendations for the subject.

### Spiral Free Drawing Test Administration

In some embodiments, an instruction screen display may include text and questions presented simultaneously with audio instruction.

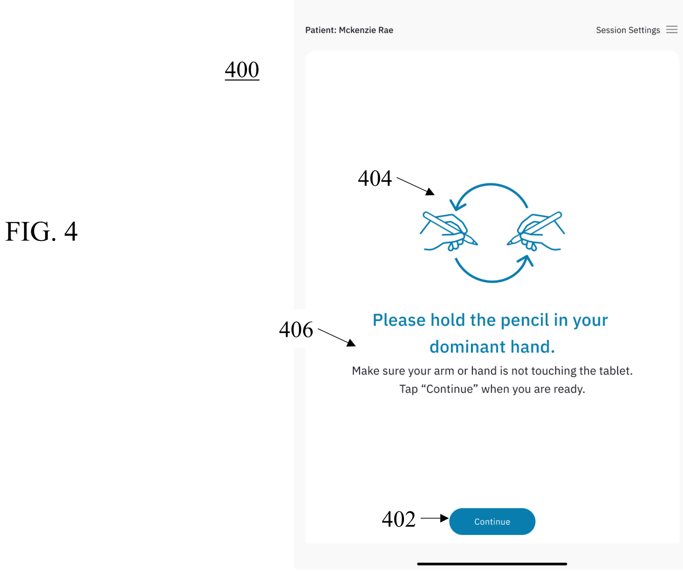

**FIG. 4** illustrates an exemplary instruction screen **400.** The instruction screen **400** includes a continue button **402** for the subject to select when ready, as indicated by the text and/or audio instructions to the subject. When the user or subject selects the continue button **402,** an animation flow may begin. The exemplary instruction screen **400** additionally includes a subject identifier, such as a name, as well as a settings menu option along the top of the screen. An exemplary graphic **404** is also shown in the center of the screen along with the instruction text **406.**

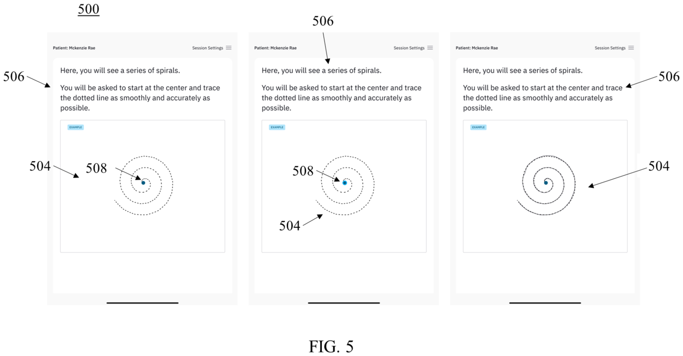

**FIGs. 5A-C** illustrate an exemplary pre-screening question and instruction display **500.** The exemplary pre-screening question and instruction display **500** as shown in **FIG. 5A** includes an example amination **504** synchronized with both displayed text instruction **506** and an audio instruction provided to the subject. The timing of the example animation The instructions explain to the subject that they will see a series of spiral drawings, and will be asked to complete tasks related to spiral tracing. As shown in **FIG. 5A,** the displayed text instruction **504** informs the patient and a dotted line spiral animation **504** is displayed. To further illustrate the task to the patient, the animation **504** proceeds to highlight the starting position **508** for the spiral. Subsequently, the animation **504** will initiate the drawing process around the spiral, as shown in **FIGs. 5B** and **5C.** The lighter blue stroke will only show during the animation **504** and not during the actual assessment. Once the spiral has been drawn completely, the subject will be automatically advanced to the practice screen.

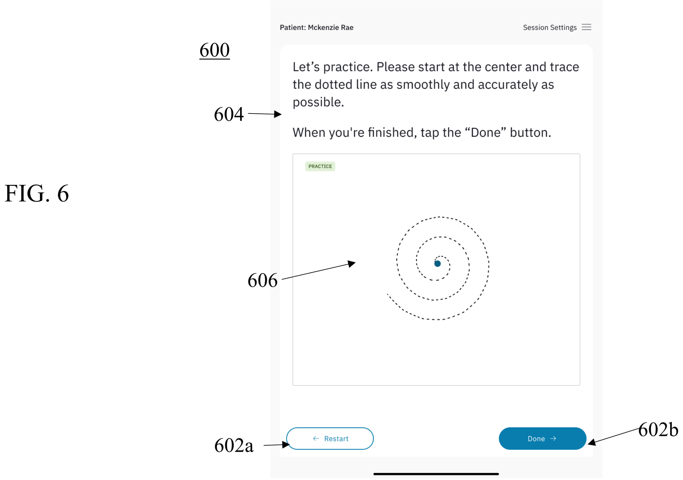

The practice screen, as illustrated in **FIG. 6****,** exhibits a user interface display **600** similar to that shown in **FIGs. 5A-5C,** albeit with distinct instructions, and is explicitly labeled as "Practice" as highlighted in the top corner of the display **600.** The subject is prompted to start tracing the spiral **606** from the center dot through both audio and displayed text instructions **604.** Drawing can be initiated at any point during the reading of the instructions or after completion. Should the device recognize that a subject is using a part of their limb to stabilize their hand during drawing, an error message will be audibly conveyed (without being displayed), advising the subject to ensure that their arm or hand does not touch the device. The verbal prompt will state to "Make sure your arm or hand is not touching the tablet." At any point, the subject has the option to tap either the "restart" button **602a** or "done" button **602b.** Choosing "restart" will return the subject to the starting point (example animation screen as shown in **FIGs. 5A-5C),** where they will go through both the example and practice phases. Selecting "done" will transition the subject to the Spiral Tracing Assessment. Subjects have the option to redo the practice by selecting a "Practice Again" button. They can restart as many times as they prefer, providing the opportunity to revisit both the example animation and the practice screen as often as necessary until they grasp the optimal way to complete the assessment.

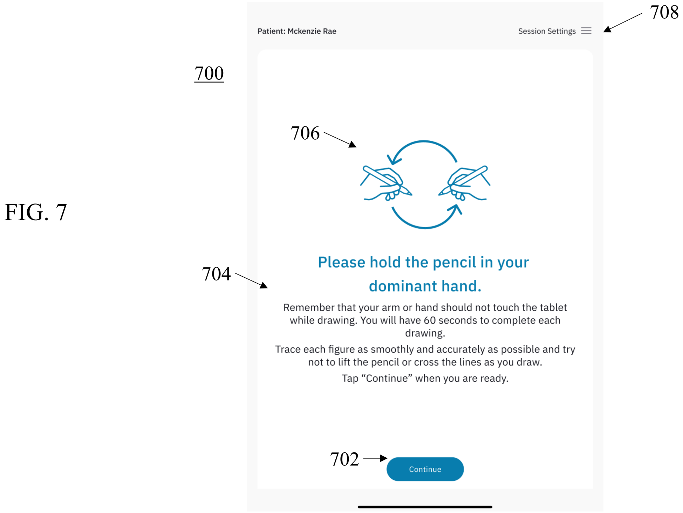

Upon completing the Practice, subjects will be automatically directed to the instruction screen **700** as shown in **FIG. 7****,** similar to the one depicted in previous figures, with additional text instructions **704** emphasizing the importance of avoiding contact between their arm or hand and the tablet while drawing as well as a 60-second time constraint for the completion of each drawing. On this screen, although there is no animation, text **704,** graphic **706,** and questions are presented simultaneously along with audio instructions. Final reminder instructions, delivered through verbal and written cues, inform the subject that they can proceed to the full assessment by pressing the "Continue" button **702.** Once on this screen, subjects cannot go back unless the Test Administrator accesses the settings menu and restarts the assessment for the subject. In the event of subject difficulties during this practice, the test administrator can use the password-protected Session Settings button **708** to choose options such as ending the session, skipping, or restarting the activity, ensuring that subjects do not accidentally activate these functions.

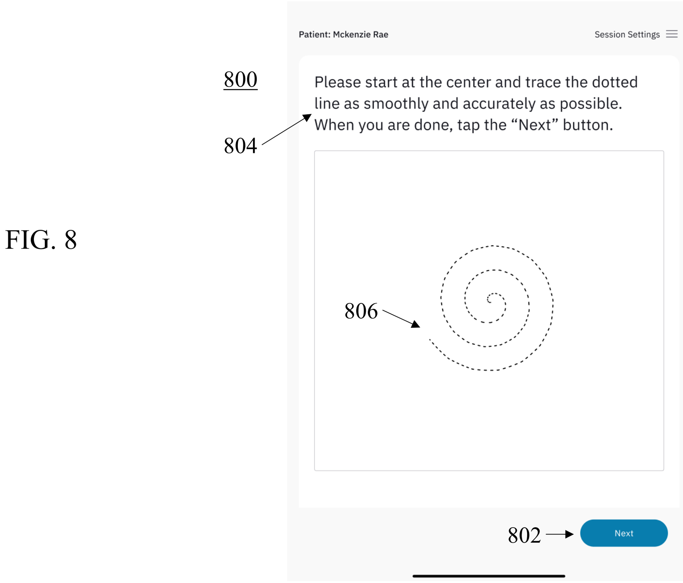

In **FIG. 8****, a** first task is explained to the subject on exemplary display screen **800.** The first task is for the subject to draw a spiral **806** with their dominant hand, in a clockwise direction. The instructions are both displayed visually in text **804** and conveyed audibly. The subject is allotted 60 seconds to complete the task. After this duration, the screen will automatically progress to the next task. Alternatively, the subject can manually advance to the next task by tapping the "Next" button **802.**

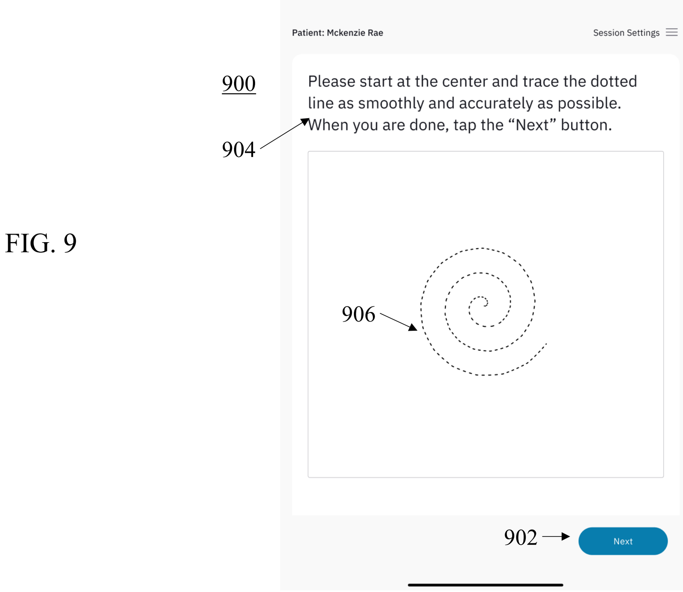

In **FIG. 9****,** a second task is outlined to the subject on exemplary display screen **900.** The second task is for the subject to draw a spiral **906** with their dominant hand, in an anti-clockwise direction. The instructions are both displayed visually in text **904** and conveyed audibly. The user is allotted 60 seconds to complete the task. After this duration, the screen will automatically progress to the next screen. Alternatively, the user can manually advance to the next screen by tapping the "Next" button **902.**

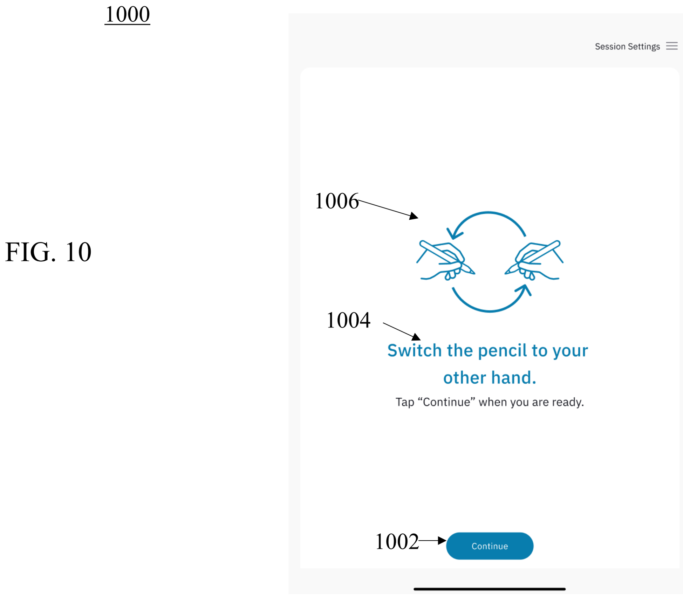

In **FIG. 10****,** a direction is given to subject on exemplary display screen **1000** to switch the pencil to the non-dominant hand. The direction is given on the display screen **1000** in text **1004,** along with a graphic **1006.** Once the subject has switched the pencil to the other hand, they can manually advance to the next screen by selecting the "Continue" button **1002.**

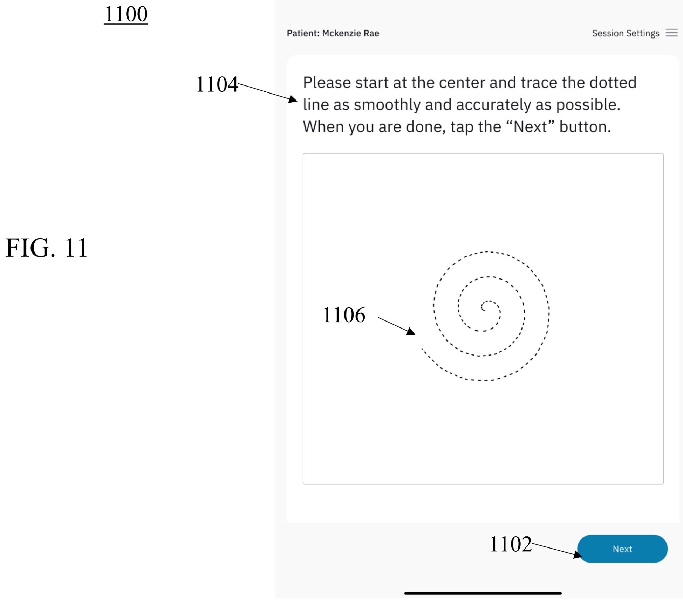

In **FIG. 11****,** a third task is explained to the subject on exemplary display screen 1100. The third task is for the subject to draw a spiral **1106** with their non-dominant hand, in the clockwise direction. The instructions are both displayed visually with text **1104** and conveyed audibly. The subject is allotted 60 seconds to complete the task. After this duration, the screen will automatically progress to the next screen. Alternatively, the subject can manually advance to the next screen by tapping the "Next" button **1102.**

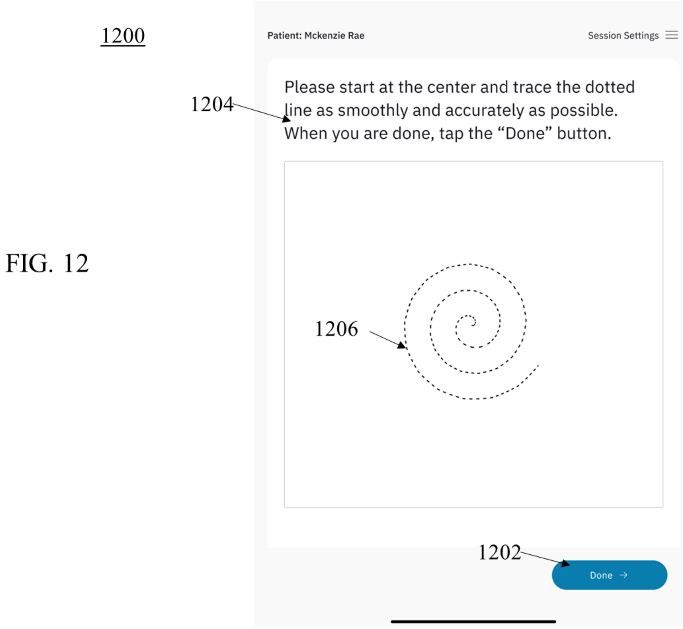

In **FIG. 12****,** a fourth task is explained to the subject on exemplary display screen **1200.** The fourth task is for the subject to draw a spiral **1206** with their non-dominant hand, in the counterclockwise direction. The instructions are both displayed visually with text **1204** and conveyed audibly. The subject is allotted 60 seconds to complete the task. After this duration, the screen will automatically progress to the completion screen. Alternatively, the user can manually advance to the completion screen by tapping the "Done" button **1202.**

### Error Handling

It is contemplated that subjects may make an error during the spiral test. These errors may be logged by the application or manually input by the test administrator. In one scenario, the subject may not understand how to perform the spiral tracing assessment. If the subject does not understand, the test administrator may manually end the test session through the session settings. The test administrator may have the opportunity to report session observations after the patient completes or times out of the assessment. This reporting is a configurable screen than can be turned on or off for organizations, clinicians, or administrators.

In an alternative scenario, the subject may place a portion of their hand or arm on the test device to aid in the drawing of the spiral. In some embodiments, the test device may detect that the subject is resting a portion of their limb on the tablet to help stabilize their hand while drawing. In some embodiments, an audio error may be read to the subject, including language such as "make sure your arm or hand is not touching the tablet." The number of times this message is read to the subject is not limited in some embodiments; in alternative embodiments, there is a limit to how many times the message is read to the subject. The message may be read to the subject during any of the segments during assessment.

In other scenarios, the subject may exceed the allotted time, thereby obtaining inaccurate and/or incomplete results.

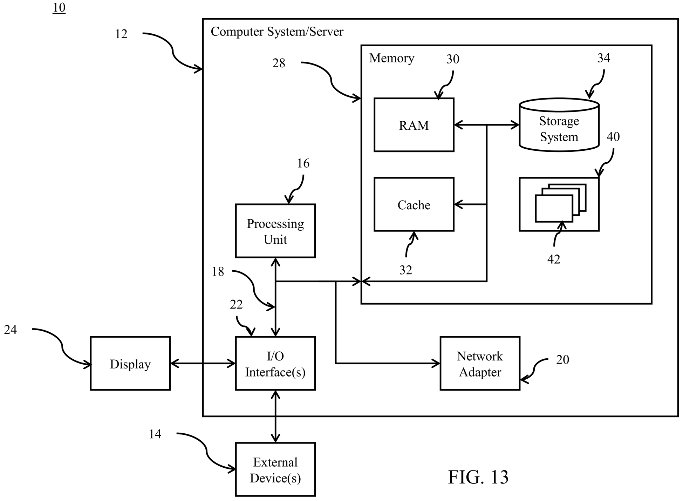

Referring now to **FIG. 13****,** a schematic of an example of a computing node is shown. Computing node **10** is only one example of a suitable computing node and is not intended to suggest any limitation as to the scope of use or functionality of embodiments described herein. Regardless, computing node **10** is capable of being implemented and/or performing any of the functionality set forth hereinabove.

In computing node **10** there is a computer system/server **12,** which is operational with numerous other general purpose or special purpose computing system environments or configurations. Examples of well-known computing systems, environments, and/or configurations that may be suitable for use with computer system/server **12** include, but are not limited to, personal computer systems, server computer systems, thin clients, thick clients, handheld or laptop devices, multiprocessor systems, microprocessor-based systems, set top boxes, programmable consumer electronics, network PCs, minicomputer systems, mainframe computer systems, and distributed cloud computing environments that include any of the above systems or devices, and the like.

Computer system/server **12** may be described in the general context of computer system-executable instructions, such as program modules, being executed by a computer system. Generally, program modules may include routines, programs, objects, components, logic, data structures, and so on that perform particular tasks or implement particular abstract data types. Computer system/server **12** may be practiced in distributed cloud computing environments where tasks are performed by remote processing devices that are linked through a communications network. In a distributed cloud computing environment, program modules may be located in both local and remote computer system storage media including memory storage devices.

As shown in **FIG. 13****,** computer system/server **12** in computing node **10** is shown in the form of a general-purpose computing device. The components of computer system/server **12** may include, but are not limited to, one or more processors or processing units **16,** a system memory **28,** and a bus **18** that couples various system components including system memory **28** to processor **16.**

Bus **18** represents one or more of any of several types of bus structures, including a memory bus or memory controller, a peripheral bus, an accelerated graphics port, and a processor or local bus using any of a variety of bus architectures. By way of example, and not limitation, such architectures include Industry Standard Architecture (ISA) bus, Micro Channel Architecture (MCA) bus, Enhanced ISA (EISA) bus, Video Electronics Standards Association (VESA) local bus, Peripheral Component Interconnect (PCI) bus, Peripheral Component Interconnect Express (PCIe), and Advanced Microcontroller Bus Architecture (AMBA).

Computer system/server **12** typically includes a variety of computer system readable media. Such media may be any available media that is accessible by computer system/server **12,** and it includes both volatile and non-volatile media, removable and non-removable media.

System memory **28** can include computer system readable media in the form of volatile memory, such as random access memory (RAM) **30** and/or cache memory **32.** Computer system/server **12** may further include other removable/non-removable, volatile/non-volatile computer system storage media. By way of example only, storage system **34** can be provided for reading from and writing to a non-removable, non-volatile magnetic media (not shown and typically called a "hard drive"). Although not shown, a magnetic disk drive for reading from and writing to a removable, non-volatile magnetic disk *(e.g.,* a "floppy disk"), and an optical disk drive for reading from or writing to a removable, non-volatile optical disk such as a CD-ROM, DVD-ROM or other optical media can be provided. In such instances, each can be connected to bus **18** by one or more data media interfaces. As will be further depicted and described below, memory **28** may include at least one program product having a set *(e.g.,* at least one) of program modules that are configured to carry out the functions of embodiments of the disclosure.

Program/utility **40,** having a set (at least one) of program modules **42,** may be stored in memory **28** by way of example, and not limitation, as well as an operating system, one or more application programs, other program modules, and program data. Each of the operating system, one or more application programs, other program modules, and program data or some combination thereof, may include an implementation of a networking environment. Program modules **42** generally carry out the functions and/or methodologies of embodiments as described herein.

Computer system/server **12** may also communicate with one or more external devices **14** such as a keyboard, a pointing device, a display **24,** etc.; one or more devices that enable a user to interact with computer system/server **12;** and/or any devices (*e.g.*, network card, modem, etc.) that enable computer system/server **12** to communicate with one or more other computing devices. Such communication can occur via Input/Output (I/O) interfaces **22.** Still yet, computer system/server **12** can communicate with one or more networks such as a local area network (LAN), a general wide area network (WAN), and/or a public network *(e.g.,* the Internet) via network adapter **20.** As depicted, network adapter **20** communicates with the other components of computer system/server **12** via bus **18.** It should be understood that although not shown, other hardware and/or software components could be used in conjunction with computer system/server **12.** Examples, include, but are not limited to: microcode, device drivers, redundant processing units, external disk drive arrays, RAID systems, tape drives, and data archival storage systems, *etc.*

The present disclosure may be embodied as a system, a method, and/or a computer program product. The computer program product may include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (*e.g.*, light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to embodiments of the disclosure. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present disclosure. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The descriptions of the various embodiments of the present disclosure have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.
The description also relates to the following items:
Item 1: A method, in particular for screening motor and cognitive impairment and/or for determining and outputting a motor and cognitive impairment status, the method comprising:
   receiving a response to a spiral tracing assessment administered to a subject or receiving data comprising a response to a spiral tracing assessment administered to a subject;
   assessing the subject's performance based on the response;
   collecting performance parameters based on the subject's performance on the spiral tracing assessment;
   providing the performance parameters and the response to a pretrained machine learning model;
   predicting, using the pretrained machine learning model, a likelihood of the patient having a cognitive impairment;
   determining a motor and cognitive impairment status of the patient based on the prediction; and
   outputting the motor and cognitive impairment status.
Item 2: The method of item 1, further comprising administering a spiral tracing assessment to a subject, in particular prior to receiving the response of a spiral assessment administered to a subject.
Item 3: The method of item 1 or 2, wherein the spiral tracing assessment comprises directing the subject to trace a spiral in a clockwise and/or anti-clockwise direction.
Item 4: The method of any one of items 1 to 3, wherein the spiral tracing assessment comprises directing the subject to trace a spiral with a dominant and/or non-dominant hand.
Item 5: The method of any one of items 1-4, wherein the spiral tracing assessment comprises directing the subject to trace a spiral with one continuous pen stroke.
Item 6: The method of any one of items 1-5, wherein the spiral tracing assessment is completed with a digital pen.
Item 7: The method of item 6, wherein collecting performance parameters comprises reading one or more digital pen metrics, the one or more digital pen metrics collected during the spiral tracing assessment.
Item 8: The method of any one of items 1-7, wherein the response to the spiral tracing assessment comprises one or more traced spirals.
Item 9. The method of item 8, further comprising determining one or more metrics based on one or more deviations between a template spiral and the one or more traced spirals.
Item 10: The method of item 9, further comprising:
   combining the one or more metrics into a composite score;
   comparing the composite score to a threshold value; and
   based on the comparison, determining a motor and cognitive impairment level of the subject.
Item 11: The method of any one of items 1-10, wherein the motor and cognitive impairment status is one of mild or moderate.
Item 12: The method of any one of items 1-10, further comprising suggesting additional testing based on the output motor and cognitive impairment status.
Item 13: The method of any one of items 1-12, wherein the motor and cognitive impairment comprises a tremor.
Item 14: The method of item 13, wherein the tremor is determined by one or more graphomotor metrics.
Item 15: The method of item 13, wherein the tremor is determined by one or more metrics derived from a digital pen.
Item 16: The method of any one of items 1-15, wherein administering a spiral tracing assessment to a subject comprises providing the spiral tracing assessment on a tablet.
Item 17: The method of any one of items 1-16, further comprising allowing the subject to complete one or more practice spiral tracing assessments.
Item 18: The method of any one of items 1-17, wherein predicting a likelihood of the patient having a cognitive impairment comprises predicting a degree of cognitive impairment.
Item 19: The method of item 18, wherein the degree of cognitive impairment comprises one of mild, moderate, or severe.
Item 20: A system for screening motor and cognitive impairment, the system comprising:
   a computing node comprising a computer readable storage medium having program instructions embodied therewith, the program instructions executable by a processor of the computing node to cause the processor to perform a method comprising any of the methods of items 1-19.
Item 21: A computer program product for screening cognitive impairment, the computer program product comprising a computer readable storage medium having program instructions embodied therewith, the program instructions executable by a processor to cause the processor to perform a method comprising of any of the methods of items 1-19.

## Claims

1. A method for screening motor and cognitive impairment, the method comprising:
administering a spiral tracing assessment to a subject, and receiving a response thereto;
assessing the subject's performance based on the response;
collecting performance parameters based on the subject's performance on the spiral tracing assessment;
providing the performance parameters and the response to a pretrained machine learning model;
predicting, using the pretrained machine learning model, a likelihood of the patient having a cognitive impairment;
determining a motor and cognitive impairment status of the patient based on the prediction; and
outputting the motor and cognitive impairment status.

2. The method of claim 1, wherein the spiral tracing assessment comprises directing the subject to trace a spiral in a clockwise and/or anti-clockwise direction.

3. The method of claim 1 or 2, wherein the spiral tracing assessment comprises directing the subject to trace a spiral with a dominant and/or non-dominant hand.

4. The method of any one of claims 1-3, wherein the spiral tracing assessment comprises directing the subject to trace a spiral with one continuous pen stroke.

5. The method of any one of claims 1-4, wherein the spiral tracing assessment is completed with a digital pen.

6. The method of claim 5, wherein collecting performance parameters comprises reading one or more digital pen metrics, the one or more digital pen metrics collected during the spiral tracing assessment.

7. The method of any one of claims 1-6, wherein the response to the spiral tracing assessment comprises one or more traced spirals.

8. The method of claim 7, further comprising determining one or more metrics based on one or more deviations between a template spiral and the one or more traced spirals.

9. The method of claim 8, further comprising:
combining the one or more metrics into a composite score;
comparing the composite score to a threshold value; and
based on the comparison, determining a motor and cognitive impairment level of the subject.

10. The method of any one of claims 1-9, wherein the motor and cognitive impairment status is one of mild or moderate.

11. The method of any one of claims 1-9, further comprising suggesting additional testing based on the output motor and cognitive impairment status.

12. The method of any one of claims 1-11, wherein the motor and cognitive impairment comprises a tremor,
wherein, optionally, the tremor is determined by one or more graphomotor metrics, or
wherein, optionally, the tremor is determined by one or more metrics derived from a digital pen.

13. The method of any one of claims 1-12, wherein
administering a spiral tracing assessment to a subject comprises providing the spiral tracing assessment on a tablet, and/or
wherein the method further comprises allowing the subject to complete one or more practice spiral tracing assessments, and/or
wherein predicting a likelihood of the patient having a cognitive impairment comprises predicting a degree of cognitive impairment, wherein, optionally, the degree of cognitive impairment comprises one of mild, moderate, or severe.

14. A system for screening motor and cognitive impairment, the system comprising:
a computing node comprising a computer readable storage medium having program instructions embodied therewith, the program instructions executable by a processor of the computing node to cause the processor to perform a method comprising any of the methods of claims 1-13.

15. A computer program product for screening cognitive impairment, the computer program product comprising a computer readable storage medium having program instructions embodied therewith, the program instructions executable by a processor to cause the processor to perform a method comprising of any of the methods of claims 1-13.
